# FASCICULE DE BREVET EUROPEEN

(11) **EP 0 256 984 B1**
(45) Date de publication et mention de la délivrance du brevet: **01.03.1995**
(21) Numéro de dépôt: 87810452.0
(22) Date de dépôt: 10.08.1987
(51) Int. Cl.: A61B 17/60

(54) **Fixateur externe à biocompression pour ostéosynthèse**
Vorrichtung zur äusseren Fixierung von Knochen mit Biokompression
External bone fixation device with biocompression

(30) Priorité: 11.08.1986 ES 8603667
(43) Date de publication de la demande: 24.02.1988
(73) Titulaire: JAQUET ORTHOPEDIE S.A., CH-1228 Plan-les-Ouates (CH)
(72) Inventeur: Lazo-Zbikowski, Juan, Dr., E-41011 Sevilla (ES)
(74) Mandataire: Dietlin, Henri

(56) Documents cités:
- EP-A- 0 024 256
- EP-A- 0 099 289
- FR-A- 2 129 735
- FR-A- 2 517 535
- US-A- 2 391 537

## Description

La présente invention est du domaine de l'ostéosynthèse et a plus précisément pour objet un fixateur externe à biocompression.

On connaît depuis longtemps des fixateurs externes permettant de maintenir des fils ou des fiches transcutanées insérés dans les os de part et d'autre de la fracture au moyen de cadres extérieurs et de barres de fixation.

On a pourtant constaté que dans certains cas, en utilisant des systèmes fixes, permettant pourtant une stabilité excellente, il y avait des risques de ralentissement de la consolidation et de mauvaise formation du cal osseux.

C'est pour pallier ces inconvénients que l'on a proposé d'utiliser la "biocompression". La biocompression est un terme appliqué aux contraintes (telles que tractions et compressions) ayant leur origine dans la déformation d'un os sous des charges d'activités fonctionnelles. Les phénomènes de biocompression sont aussi appelés, selon les auteurs, activité fonctionnelle, stimuli mécaniques, compression axiale, transmission des charges ou dynamisation.

On a en effet constaté que cette biocompression est un facteur essentiel dans la consolidation d'os fracturés, qui ne réclame pas seulement une grande stabilité, qui n'est pas ostéogénique en elle-même, mais également une biocompression physiologique. En effet, lorsqu'une charge est appliquée sur un os, l'os subit une déformation (tension) et, en même temps, des forces internes (pressions) se développent, opposées à cette déformation. Des déformations élastiques ont lieu quand on retrouve l'état original après que la charge ait été supprimée. On a donc été amené à considérer les fixateurs externes sous le double angle de la rigidité et de la stabilité, la rigidité s'appliquant à l'absence d'élasticité et la stabilité étant la limitation aux déformations élastiques.

Pour réaliser cette biocompression, il faut un dispositif autorisant un glissement dans une direction parallèle à l'axe osseux, de telle manière qu'une contraction musculaire se traduise par une action de compression au niveau du foyer de la fracture entre les deux fragments de l'os, de grandeur égale et de sens opposé à la contraction. Le fixateur externe doit donc empêcher tous les déplacement angulaires, latéraux ou de rotation, et ne laisser libre que le déplacement de glissement dans la direction de l'axe longitudinal de l'os fracturé. Dans des dispositifs connus, la compression est pratiquement invariable et sa grandeur est totalement empirique.

On connaît déjà de nombreux modèles de fixateurs externes, de formes mécaniques distinctes, mais qui sont essentiellement de principe identique : plusieurs fiches ou clous de fixation, ayant une prise sur chacun des fragments de l'os fracturé, sont solidarisés entre eux extérieurement par rapport au membre, au moyen de cadres ou de barres métalliques. Ces barres sont de deux types, les barres de neutralisation et les barres de compression.

Les barres de neutralisation sont des barres de métal, généralement en acier, de diverses dimensions et formes, qui sont rendues solidaires des fiches osseuses par différents dispositifs de serrage pour constituer un ensemble fixe.

Les barres de compression sont constituées par des dispositifs à vis, blocables, qui permettent l'application d'une compression interfragmentaire permanente, constante et pratiquement invariable. Après blocage de la vis, la pression appliquée se maintient théoriquement constante pendant la période séparant deux ajustements pratiqués par le médecin. En variante, on utilise des ressorts ou des tirants de caoutchouc en remplacement des vis.

Par le brevet espagnol 483 191, on connaît un système de fixation basé sur le fait d'utiliser des barres télescopiques, à glissement relatif, formées par un double tube, le tube extérieur étant d'un diamètre plus grand et son diamètre intérieur égal au diamètre extérieur du tube intérieur. En vue de solidariser chacune de ces deux pièces télescopiques à l'un des deux fragments d'un os fracturé, on utilise une barre parallèle à l'axe principal de l'os pour produire une compression axiale au niveau de la fracture sous l'action des muscles. On utilise ainsi le fait que le muscle n'est jamais en relaxation totale et que, même si le membre est au repos, il existe une contraction permanente dénommée "tonus" musculaire. En d'autres termes, la fracture est soumise en permanence à des compressions variables provenant, dans le sens croissant :
- du tonus musculaire (à l'état de repos du membre, tout en tenant compte de la force gravitationnelle),
- des contractions musculaires dues à des gestes,
- d'une charge statique (poids du patient),
- d'une charge dynamique pendant la marche (faisant intervenir le poids multiplié par l'accélération),
toutes ces stimulations étant naturelles (d'où la notion "biologie" du préfixe "bio"), et non pas mécaniques comme celles provenant de barres de compression.

Pour éviter tout déplacement angulaire et toute rotation au niveau de la fracture, on utilise généralement deux barres télescopiques, disposées de part et d'autres des fiches transfixiantes, c'est-à-dire de fiches traversant le membre de part en part, comme décrit dans la demande de brevet européen EP-A-0.999.289. Comme l'axe de coulissement doit être disposé dans une direction parallèle à l'axe longitudinal de l'os, ces barres sont maintenues au moyen de dispositifs de serrage tels que des mâchoires orientables ou rotules de liaison, par exemple celles décrites dans la demande de brevet EP-A-0.024.256 qui propose un appareil d'allongement ou de compression des fragments osseux.

Le brevet français 2.517.535 décrit un dispositif d'immobilisation fonctionnelle pour ostéosynthèse au moyen d'organes fixateurs extérieurs qui assurent une réunion des moyens de maintien des clous de fixation et d'ancrage osseux dans les fragments, de part et d'autre de la fracture, au moyen de barres coulissant télescopiquement jouant le rôle de biocompresseurs. Ces barres sont réunies aux clous de fixation au moyen de mâchoires, rotules ou autres dispositifs de serrage, les stimuli mécaniques de compression dans le foyer, d'amplitude physiologique de même intensité que le tonus musculaire et variables selon l'activité fonctionnelle, la charge statique et la charge dynamique étant utilisés en tant que système d'immobilisation fonctionnelle.

Dans ce dispositif la barre mobile de l'élément télescopique est munie d'une surface plane longitudinale sur une partie de sa longueur et la barre fixe est solidaire d'un corps dans lequel coulisse la barre mobile et qui présente une surface coopérant avec ladite surface plane longitudinale, pour éviter tout déplacement angulaire.

Ce brevet décrit un système comportant plusieurs barres de biocompression, de manière à assurer la stabilité par rapport à la rotation. Il a donc pour inconvénient d'être encombrant pour le patient, puisque le cadre de maintien s'étend de chaque côté des fiches transfixiantes, c'est-à-dire de chaque côté du membre fracturé.

Le but de la présente invention est de proposer un dispositif dont la grandeur de la compression est variable en fonction de paramètres tels que le tonus musculaire (au repos), la contraction musculaire (lors de mouvements), le poids de l'individu (au repos) ainsi que la charge dynamique (marche et poids par accélération). Ce fixateur externe a de plus l'avantage d'être le seul lien entre les groupes de fiches disposés de part et d'autre de la fracture, diminuant ainsi le poids et l'encombrement du dispositif pour le patient.

Il présente de plus l'avantage d'être très rapidement mis en place et réglé, ce qui est d'une grande importance si l'on tient compte du fait que l'opération de mise en place a lieu sous anesthésie et que la durée de celle-ci sera par conséquent considérablement réduite.

Le fixateur externe selon l'invention comporte deux groupes d'au moins une fiche maintenant chacun un fragment d'os et un élément télescopique composé d'une barre mobile coulissant sans déplacement angulaire dans une barre fixe afin d'autoriser un mouvement de va-et-vient dit de biocompression, chaque groupe de fiches étant solidaire de l'une des barres mobile et fixe, dans lequel :
- la barre mobile est munie d'au moins une surface plane longitudinale sur une partie de sa longueur,
- la barre fixe est solidaire d'un corps dans lequel coulisse la barre mobile, ledit corps présentant au moins une surface coopérant avec la surface plane longitudinale pour éviter tout déplacement angulaire.

Il est caractérisé en ce que des moyens de roulement sont intercalés entre l'intérieur du corps et la barre mobile et en ce que le corps comporte une pièce de guidage présentant une surface coopérant avec la surface plane longitudinale.

Les moyens de roulement destinés à diminuer la friction sont réalisés de différentes manières. Avec un revêtement, constitué par exemple de microbilles métalliques enrobées dans un revêtement plastique. En variante, on pourra utiliser également un roulement à aiguilles, par exemple de section carrée, et consistant en une cage dont chaque côté est constitué par un séparateur présentant une succession de dégagements destinés à recevoir chacun une aiguille.

En variante les moyens de roulement sont constitués par une douille à billes, disposée entre deux râcleurs. Cette douille à billes est disposée entre la barre mobile et un corps de guidage solidaire de la barre fixe.

Pour assurer aisément le parallélisme entre l'os fracturé et l'élément télescopique, l'une des barres peut présenter une partie coudée.

Le dessin annexé représente, à titre d'exemples non limitatifs, deux formes d'exécution de l'objet de la présente invention.

La figure 1 est une vue générale en élévation montrant schématiquement un fixateur selon une première forme d'exécution, ainsi que les moyens d'ancrage dans l'os fracturé.

Les figures 2 et 3 sont des agrandissements partiels du fixateur schématisé à la figure 1, respectivement vu de côté et de dessus.

La figure 4 est une coupe longitudinale de ce même appareil, encore légèrement agrandi, afin de préciser ses composants principaux.

Les figures 5, 6 et 7 sont des coupes transversales selon V-V, VI-VI et VII-VII respectivement aux figures 2 ou 3 et présentant respectivement le système de fixation d'une des barres, le système de guidage de la barre mobile ainsi que l'embout de montage.

La figure 8 est une vue en perspective de l'appareil selon l'invention, dans une seconde forme d'exécution.

La figure 9 est une vue de l'appareil de la figure 8 auquel on a enlevé le couvercle du corps principal représenté partiellement en coupe.

La figure 10 est une vue en coupe longitudinale de l'appareil des figures 8 et 9 tandis que la figure 11 est une coupe transversale, selon XI-XI à la figure 10.

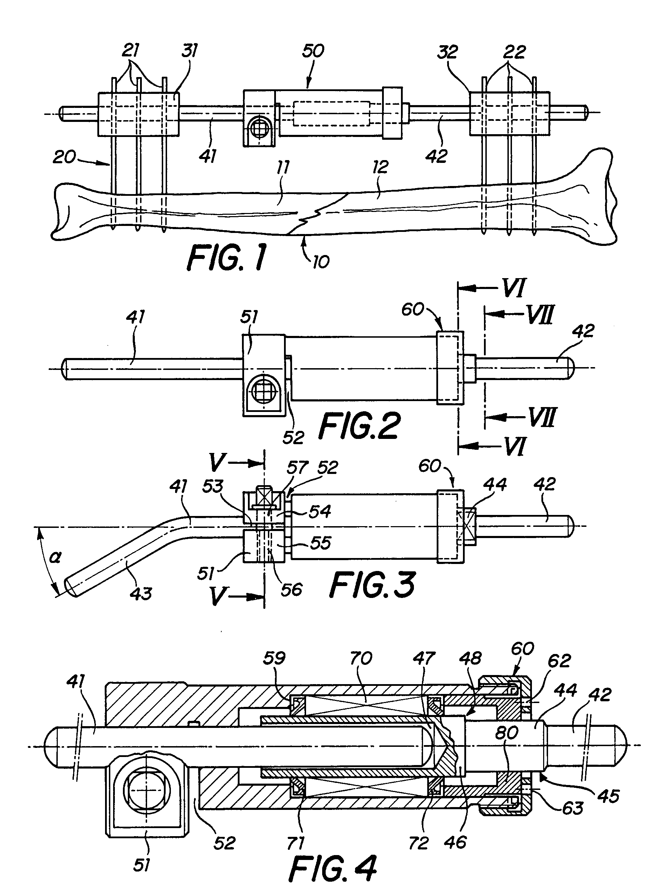

Dans la représentation schématique de la figure 1, on a représenté un os 10 fracturé en deux éléments 11 et 12. Dans chacun des éléments osseux, on distingue des groupes de clous ou fiches 20. A la figure 1 on a représenté plus particulièrement un premier groupe de trois fiches 21 introduites dans l'élément 11 et un second groupe de fiches 22 insérées dans l'élément osseux 12.

Chaque groupe de fiches 21 ou 22 est maintenu dans un élément de liaison correspondant 31 ou 32, par exemple constitué par un étau donc chaque mâchoire comporte des rainures destinées au positionnement et au maintien des fiches 21 et 22. Ces éléments de liaison 31 ou 32 sont d'autre part également solidaires des barres 41 et 42 destinées à assurer la liaison entre les deux groupes de fiches 21 et 22, par l'intermédiaire d'un corps télescopique 50. Il est bien sûr possible de remplacer les éléments de liaison 31 et 32 par des rotules orientables, selon chaque emploi spécifique.

Les figures 2 et 3 présentent le corps télescopique 50 agrandi ainsi que les barres précédemment décrites, la barre 41 étant une barre fixe et la barre 42 une barre mobile. La partie arrière 51 du corps 50 est destinée à la fixation de la barre 41, tandis que sa partie avant est fermée par un embout 60, vissé à l'extérieur du corps 50 et configuré pour laisser libre passage à la barre mobile 42. On remarquera à la figure 3 que la barre 41 présente un coude d'angle α, généralement compris entre 20° et 90°, au-delà duquel la partie arrière 43 est rectiligne.

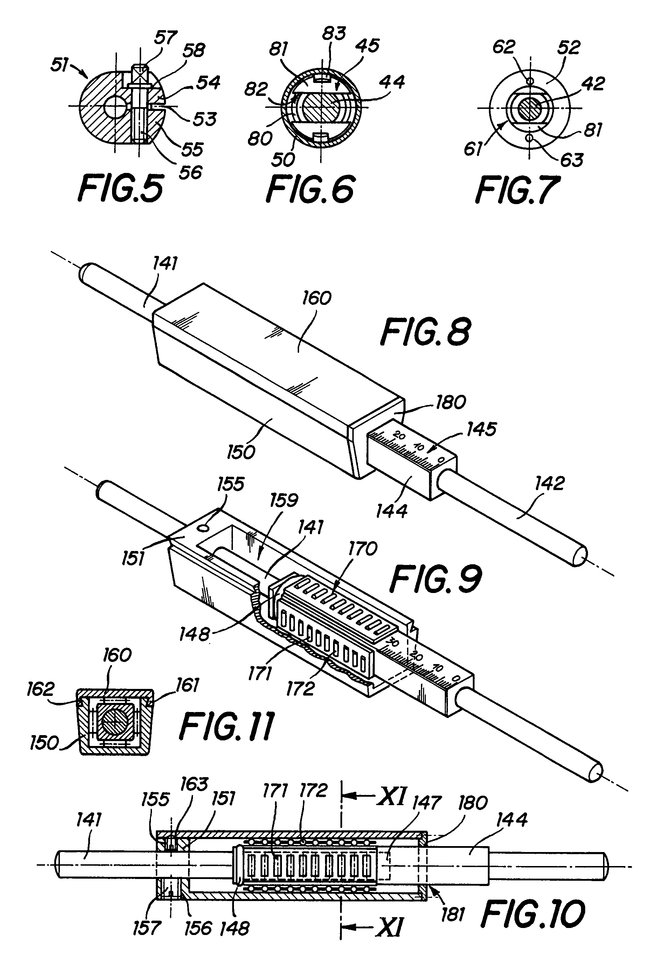

L'arrière 51 présente une fente transversale 52 et une fente longitudinale 53 destinées à créer deux ailes 54 et 55, légèrement déformables pour permettre le serrage de la barre 41. Comme visible à la coupe de la figure 5, l'aile 55 est traversée perpendiculairement à la fente 53 par une ouverture taraudée 56 destinée à recevoir une vis 57, traversant librement une ouverture correspondante pratiquée dans l'aile 54 et dont la tête vient s'appuyer sur le fond d'un dégagement 58 prévu à cet effet. Il est à noter que la vis 57 présente une tête carrée destinée à coopérer avec une clé de serrage correspondante et qui, en position de fixation, est pratiquement noyée dans la masse du corps 50, en raison du dégagement 58.

La barre 42 présente une partie médiane 44, d'un diamètre supérieur à celui de la barre 42, munie de deux plats longitudinaux 45, parallèles, comme visible à la figure 6, et destinés au guidage de la barre mobile, pour empêcher sa rotation.

En revenant à la figure 4, on remarque que la partie arrière 46 présente une ouverture centrale 47 cylindrique, de dimension légèrement supérieure au diamètre de la barre 41. Le diamètre extérieur de la partie arrière 46 est supérieur à celui de la partie médiane 44, de manière à créer un épaulement 48 de butée.

Bien que pour la clarté de l'exposé on ait subdivisé la barre mobile en parties distinctes 42, 44 et 46, il ne s'agit que d'une seule pièce, obtenue par usinage ou éventuellement par soudage des parties distinctes.

La partie arrière 46 de la barre mobile coopère avec une douille à billes 70 destinée à faciliter le glissement relatif entre les deux barres 41 et 42 et plus précisément entre le corps 50, solidaire de la barre 41, et la barre 42. La douille à billes 70 est insérée dans une ouverture centrale correspondante présentant un rebord 59 de butée et elle est fixée entre deux racleurs 71 et 72.

Elle coopère d'autre part avec une pièce de guidage 80 se présentant sous la forme d'un cylindre limité sur une face par une collerette 81, d'un diamètre supérieur à celui du cylindre 80. La collerette 81 est dimensionnée pour s'appuyer en bout du corps 50, tandis que sa partie cylindrique 80 s'engage dans l'ouverture du corps 50. La collerette 81 est traversée par un fraisage 82 de largeur correspondant à la distance entre les deux plats longitudinaux 45 de la partie médiane 44 de la barre mobile. La pièce de guidage 80 est rendue solidaire du corps 50 grâce à deux évidements 83 destinés à coopérer avec des languettes correspondantes du corps 50.

La pièce de guidage 80 est fixée dans le corps 50 au moyen d'un embout 60, configuré pour se visser à l'extérieur du corps 50. L'embout 60 présente une ouverture circulaire 61, destinée au passage de la partie médiane 44 de la barre mobile. Il est d'autre part muni de deux trous opposés 62 et 63, destinés à recevoir deux picots d'une clé de serrage permettant de bloquer le montage des différents éléments mentionnés jusqu'ici.

Pour limiter le poids du fixateur, on choisira de réaliser le corps 50, son embout 60 ainsi que le guide 80 en alliage léger, par exemple en aluminium.

Pour des questions de rigidité de l'ensemble, les tiges fixe et mobile 41 et 42 seront de préférence en acier inoxydable. Il est à noter que tous les matériaux utilisés sont aptes à la stérilisation.

Dans la variante présentée aux figures 8 à 11, on retrouve une barre fixe 141 et une barre mobile 142 présentant une partie'médiane 144 de section carrée et de dimensions supérieures au diamètre de la barre 142 et destinée à coulisser par rapport au corps 150 et à son couvercle 160. Ce couvercle comporte deux bords latéraux 161 et 162, destinés à coopérer avec des dégagements correspondants pratiqués dans le corps 150 (fig. 11).

On remarquera que la partie médiane 144 comporte une échelle graduée 145 permettant de mesurer le mouvement relatif. Bien que cette échelle n'ait pas figuré dans la première version décrite, il va de soi qu'elle peut exister également.

La seconde variante se distingue essentiellement par le fait que la partie médiane 144 de la barre mobile est de section carrée. Cette partie est destinée à glisser dans un roulement à aiguilles 170 constitué d'une cage prismatique de section carrée, dont chaque côté est constitué par un séparateur présentant une succession de dégagements rectangulaires 171 destinés à recevoir chacun une aiguille 172. La partie médiane 144 de la barre mobile présente un évidement central 147 destiné au passage de la barre fixe 141, qui comporte une plaquette de butée 148.

Le roulement à aiguilles 170 est disposé dans un dégagement correspondant 159 réalisé dans le corps 150 et particulièrement visible à la figure 9, lorsque le couvercle 160 est retiré. On remarque que le corps 150 a la forme d'un tube profilé en U, dont l'une des extrémités est fermée par une paroi fixe 151, tandis que l'autre extrémité se termine par une plaquette 180 présentant une ouverture centrale 181 de forme carrée destinée au passage de la barre carrée 144. Le couvercle 160 est apte à coulisser le long du corps 150 par rapport auquel il est maintenu au moyen d'une vis 163 insérée dans un dégagement correspondant 155 pratiqué dans la paroi fixe 151. La barre fixe 141 est rendue solidaire du corps 150 au moyen d'une vis 157 pénétrant dans le taraudage 156 pratiqué à cet effet dans la paroi 151.

On a représenté au dessin un roulement à aiguilles 170 de section carrée, mais sans sortir du cadre de la présente invention on peut prévoir de le remplacer par une section polygonale différente, par exemple triangulaire, rectangulaire ou hexagonale.

Les barres fixe 141 et mobile 142 et 144 sont généralement en acier inoxydable, mais d'autres alliages sont également possibles, tels que ceux au Cr-Co-Mo ou au titane qui présentent d'excellentes qualités mécaniques et ont un poids relativement faible.

Les séparateurs constituant la cage 170 peuvent également être constitués de matériaux de type plastique, tels que polyéthylène à haute densité, nylon, teflon ou autres, à conditions que leurs caractéristiques mécaniques soient appropriées et qu'ils soient résistants à des températures de stérilisation en vapeur saturée, pouvant s'élever jusqu'à 140°C.

Comme on l'a déjà dit, le fixateur externe doit être mis en place au cours d'une opération ayant lieu sous anesthésie. Il est donc particulièrement intéressant d'avoir à sa disposition un corps télescopique monté, permettant de réduire la durée de l'anesthésie.

Dans l'exemple des figures 8 à 10, on disposera la barre carrée 144, entourée de sa cage à aiguilles 170 dans le dégagement 159 pratiqué dans le corps 150. On glissera les bords latéraux 161 et 162 du couvercle 160 dans les dégagements correspondants du corps 150 et on fixera le couvercle 160 par rapport au corps 150 au moyen de la vis 163 insérée dans le taraudage correspondant 155, après l'avoir engagée dans l'ouverture 156 prévue pour la vis 157 de fixation de la barre fixe. On fixera par tous moyens la plaquette avant 180 et on fixera la barre fixe 141 au moyen de la vis 157.

Dans la forme d'exécution présentée aux figures 2 à 7, on montera la douille à bille 70, munie de chaque côté de ses racleurs 71 et 72, autour de la partie arrière 46 de la barre mobile et on l'engagera dans le corps 50 en appui sur le rebord de butée 59 du dégagement central. On introduira le guide 80 autour de la partie médiane 44 de la barre mobile, pour empêcher tout mouvement de rotation de la barre mobile 42 par rapport au corps 50, en alignant les plats longitudinaux 45 dans les dégagements correspondants du fraisage 82, et on serrera l'embout 60 au moyen d'un outil spécial muni de pitons destinés à s'engager dans les trous 62 et 63.

Il est à remarquer que, quelle que soit la forme d'exécution, le dispositif télescopique monté peut être soumis aux traitements de stérilisation.

Selon l'utilisation particulière on choisira la longueur et la forme de la barre fixe 41 en fonction de l'os dans lequel on utilise le système et en fonction de la distance à laquelle les fiches seront insérées dans les fragments osseux.

Quant à la mise en place au cours de l'opération, elle est la suivante. Le médecin introduit d'abord les fiches 21 et 22 dans les fragments osseux 11 et 12 situés de part et d'autre de la fracture. Il dispose alors l'élément télescopique 50 ou 150 parallèlement à l'axe principal de l'os fracturé 10, au moyen des étaux de fixation 31 et 32.

Pour assurer que le déplacement dû à la biocompression ait lieu selon un axe parallèle à l'axe longitudinal de l'os fracturé, le praticien solidarise la barre fixe 41 ou 141 à la partie arrière 51 ou 151 du corps télescopique 50 ou 150, en serrant la vis 57 ou 157 dans le taraudage 56 ou 156 correspondant.

En revenant à la figure 3 qui représente une barre 41 présentant une partie arrière 43 coudée, on voit avec quelle facilité on peut assurer rapidement l'alignement axial de l'élément télescopique avec l'os fracturé, par rotation de l'élément télescopique.

Le mouvement en compression est limité par la butée de l'extrémité de la barre fixe 41 dans l'ouverture centrale 47 de la barre mobile tandis que le mouvement en extension est limité par l'épaulement 48 de butée sur l'intérieur du guide 80.

## Revendications

1. Fixateur externe comportant deux groupes d'au moins une fiche (21,22) maintenant chacun un fragment d'os (11,12) et un élément télescopique composé d'une barre mobile (42,44; 142,144) coulissant sans déplacement angulaire dans une barre fixe (41;141) afin d'autoriser un mouvement de va-et-vient dit de biocompression, chaque groupe de fiches étant solidaire de l'une des barres mobile et fixe, dans lequel :
- la barre mobile est munie d'au moins une surface plane longitudinale (45;145) sur une partie de sa longueur,
- la barre fixe (41;141) est solidaire d'un corps (50;150, 160) dans lequel coulisse la barre mobile (42,44;142,144), ledit corps présentant au moins une surface coopérant avec la surface plane longitudinale (45;145) pour éviter tout déplacement angulaire,
caractérisé en ce que des moyens de roulement (70;170) sont intercalés entre l'intérieur du corps et la barre mobile et en ce que le corps comporte une pièce de guidage (80;180) présentant une surface (82;181) coopérant avec la surface plane longitudinale (45;145).

2. Fixateur externe selon la revendication 1, caractérisé en ce qu'une desdites barres présente une partie coudée d'un angle α dans le but de faciliter l'alignement des moyens de roulement et de l'axe longitudinal de l'os fracturé.

3. Fixateur externe selon la revendication 2, caractérisé en ce que l'angle α est compris entre 0° et 90°.

4. Fixateur externe selon la revendication 1, caractérisé en ce que l'une au moins desdites barres comporte une échelle graduée (145).

5. Fixateur externe selon la revendication 1, caractérisé en ce que lesdites barres sont en acier inoxydable, en alliage au titane ou en alliage au chrome-cobalt-molybdène.

6. Fixateur externe selon la revendication 1, caractérisé en ce que les moyens de roulement sont constitués par un revêtement.

7. Fixateur externe selon la revendication 6, caractérisé en ce que ledit revêtement est constitué par des microbilles métalliques enrobées dans un revêtement plastique.

8. Fixateur externe selon la revendication 1, caractérisé en ce que les moyens de roulement sont constitués par un roulement à aiguilles (170).

9. Fixateur externe selon la revendication 8, caractérisé en ce que le roulement à aiguilles (170) présente une section carrée.

10. Fixateur externe selon la revendication 9, caractérisé en ce que le roulement à aiguilles consiste en une cage prismatique de section carrée, dont chaque côté est constitué par un séparateur présentant une succession de dégagements rectangulaires (171) destinés à recevoir chacun une aiguille (172).

11. Fixateur selon la revendication 10, caractérisé en ce que les aiguilles sont en acier.

12. Fixateur selon la revendication 10, caractérisé en ce que les séparateurs sont en plastiques.

13. Fixateur externe selon la revendication 1, caractérisé en ce que les moyens de glissement par roulement sont constitués par une douille à billes (70).

14. Fixateur externe selon la revendication 13, caractérisé en ce que la douille à billes est disposée entre deux râcleurs (71,72).

15. Fixateur externe selon la revendication 1, caractérisé par le fait que ladite pièce de guidage est constituée par un embout (60,80;180) apte à délimiter une ouverture de forme correspondant à celle de la barre (44;144).

16. Fixateur externe selon la revendication 15, caractérisé par le fait que le corps et l'embout sont en alliage léger, en alliage est à base d'aluminium ou en matières plastiques.

## Claims

1. An external fixator comprising two groups of at least one pin (21,22), each maintaining a bone fragment (11,12), and a telescopic piece comprising a a movable bar (42,44; 142,144) which can longitudinally slide with respect to a fixed bar (41;141) without any angular displacement, in order to authorize a reciprocating movement named biocompression movement, each pin group being connected to one of the fixed or movable bar, in which:
- the movable bar possesses at least a longitudinal flat surface (45;145) on a part of its length,
- the fixed bar (41;141) is attached to a member (50; 150,160) in which the movable bar (42,44; 142,144) slides, said member presenting at least a surface cooperating with said longitudinal flat surface (45;145), to avoid any angular displacement,
characterized in that a rolling means (70;170) is inserted between the internal part of the member and the movable bar and in that the member comprises a guiding element (80;180) which presents a surface (82;181) cooperating with the longitudinal flat surface (45;145).

2. An external fixator according to claim 1, characterized in that one of said bars presents an angulated end portion (43) defining an angle α in order to easily aline said rolling means with the longitudinal axis of the fractured bone.

3. An external fixator according to claim 2, characterized in that the angle α is comprised between 0 and 90°.

4. An external fixator according to claim 1, characterized in that one at least of said bars presents a graduate scale (145).

5. An external fixator according to claim 1, characterized in that said bars are made of stainless steel, of a titanium alloy or a Cr-Co-Mo containing alloy.

6. An external fixator according to claim 1, characterized in that said rolling means is a low frictional coating.

7. An external fixator according to claim 6, characterized in that said coating comprises metallic microballs wrapped up in a plastic cover.

8. An external fixator according to claim 1, characterized in that said rolling means is a needle bearing (170).

9. An external fixator according to claim 8, characterized in that said needle bearing (170) presents a square cross section.

10. An external fixator according to claim 9, characterized in that said needle bearing is constituted by a prismatic casing, each side of which is made of a separator presenting a succession of rectangular openings (171) fitted to receive a needle (172) each.

11. An external fixator according to claim 10, characterized in that said needles are made of steel.

12. An external fixator according to claim 10, characterized in that said separators are made of plastics.

13. An external fixator according to claim 1, characterized in that said rolling means is a ball bushing (70).

14. An external fixator according to claim 13, characterized in that said ball bushing is fitted between two scraper rings (71,72).

15. An external fixator according to claim 1, characterized in that said guiding element is constituted by a tip (60,80;180) presenting an opening in the shape of the bar (44;144).

16. An external fixator according to claim 15, characterized in that the piece and the tip are made of a light metal alloy, an alloy including aluminum or plastic materials.

## Patentansprüche

1. Äußere Fixiervorrichtung, mit zwei Gruppen mit wenigstens einem Stift (21, 22), die jedes ein Knochenfragment (11, 12) halten, und einem teleskopischen Element, das aus einer beweglichen Stange (42, 44; 142, 144), welche ohne winkelmäßige Verlagerung in einer festen Stange (41; 141) gleitet, um eine Hin- und Herbewegung zu ermöglichen, die Biokompression genannt wird, besteht, wobei jede Gruppe von Stiften einstückig mit entweder der beweglichen oder festen Stange ausgebildet ist, in der:
- die bewegliche Stange mit wenigstens einer ebenen Längsfläche (45; 145) auf einem Teil seiner Länge versehen ist,
- die feste Stange (41; 141) einstückig mit einem Körper (50; 150, 160) ausgebildet ist, in der die bewegliche Stange (42, 44; 142, 144) gleitet, wobei der Körper wenigstens eine Fläche aufweist, die mit der ebenen Längsfläche (45; 145) zusammenwirkt, um jegliche winkelmäßige Verlagerung zu verhindern,
dadurch gekennzeichnet, daß Lagereinrichtungen (70; 170) zwischen das Innere des Körpers und die bewegliche Stange geschaltet sind und daß der Körper ein Führungsteil (80; 180) aufweist, das eine Fläche (82; 181) umfaßt, welche mit der ebenen Längsfläche (45; 145) zusammenwirkt.

2. Äußere Fixiervorrichtung nach Anspruch 1, dadurch gekennzeichnet, daß eine der Stangen ein Teil aufweist, das um einen Winkel o abgewinkelt ist, zu dem Zweck, die Ausrichtung der Lagereinrichtung und der Längsachse des gebrochenen Knochens zu vereinfachen.

3. Äußere Fixiervorrichtung nach Anspruch 2, dadurch gekennzeichnet, daß der Winkel o zwischen 0° und 90° liegt.

4. Äußere Fixiervorrichtung nach Anspruch 1, dadurch gekennzeichnet, daß wenigstens eine der Stangen eine Abstufung (145) aufweist.

5. Äußere Fixiervorrichtung nach Anspruch 1, dadurch gekennzeichnet, daß die Stangen aus rostfreiem Stahl, aus Titanlegierung oder aus Chrom-Kobalt-Molybdän-Legierung sind.

6. Äußere Fixiervorrichtung nach Anspruch 1, dadurch gekennzeichnet, daß die Lagereinrichtungen durch einen Überzug gebildet sind.

7. Äußere Fixiervorrichtung nach Anspruch 6, dadurch gekennzeichnet, daß der Überzug durch metallische Mikrokugeln gebildet ist, die in einen Kunststoffüberzug eingekleidet sind.

8. Äußere Fixiervorrichtung nach Anspruch 1, dadurch gekennzeichnet, daß die Lagereinrichtungen durch ein Nadellager (170) gebildet sind.

9. Äußere Fixiervorrichtung nach Anspruch 8, dadurch gekennzeichnet, daß das Nadellager (170) einen eckigen Querschnitt zeigt.

10. Äußere Fixiervorrichtung nach Anspruch 9, dadurch gekennzeichnet, daß das Nadellager aus einem prismatischen Käfig mit eckigem Querschnitt besteht, bei dem jede Seite durch eine Abtrennung gebildet ist, die eine Abfolge von rechteckigen Ausnehmungen (171) aufweist, dazu bestimmt, jeweils eine Nadel (172) aufzunehmen.

11. Fixiervorrichtung nach Anspruch 10, dadurch gekennzeichnet, daß die Nadeln aus Stahl sind.

12. Fixiervorrichtung nach Anspruch 10, dadurch gekennzeichnet, daß die Abtrennungen aus Kunststoff sind.

13. Äußere Fixiervorrichtung nach Anspruch 1, dadurch gekennzeichnet, daß die Wälzgleiteinrichtungen durch eine Kugelhülse (70) gebildet sind.

14. Äußere Fixiervorrichtung nach Anspruch 13, dadurch gekennzeichnet, daß die Kugelhülse zwischen zwei Streifringen (71, 72) angeordnet ist.

15. Äußere Fixiervorrichtung nach Anspruch 1, dadurch gekennzeichnet, daß das Führungsteil durch einen Ansatz (60, 80; 180) gebildet ist, dazu ausgelegt, eine Öffnung mit einer Form entsprechend der der Stange (44; 144) zu begrenzen.

16. Äußere Fixiervorrichtung nach Anspruch 15, dadurch gekennzeichnet, daß der Körper und der Ansatz aus einer Leichtlegierung sind, wobei die Legierung auf Aluminiumbasis ist, oder aus Kunststoff.
